# EUROPÄISCHE PATENTSCHRIFT

(11) **EP 2 244 628 B1**
(45) Veröffentlichungstag und Bekanntmachung des Hinweises auf die Patenterteilung: **29.05.2019**
(21) Anmeldenummer: 09713862.2
(22) Anmeldetag: 24.02.2009
(51) Int. Cl.: A61B 5/00

(54) **PATIENTENDATEN-SENSORVORRICHTUNG**
PATIENT DATA SENSOR DEVICE
DISPOSITIF DE DÉTECTION DE DONNÉES DE PATIENT

(30) Priorität: 28.02.2008 DE 102008011601
(43) Veröffentlichungstag der Anmeldung: 03.11.2010
(73) Patentinhaber: RAUMEDIC AG, 95213 Münchberg (DE)
(72) Erfinder: GÖHLER, Karlheinz, 08297 Zwönitz (DE); KUNZE, Gerd, 08297 Zwönitz (DE); VON FALKENHAUSEN, Christian, 53340 Meckenheim (DE)
(74) Vertreter: Rau, Schneck & Hübner Patentanwälte Rechtsanwälte PartGmbB
(86) Internationale Anmeldenummer: PCT/EP2009/001284
(87) Internationale Veröffentlichungsnummer: WO 2009/106284

(56) Entgegenhaltungen:
- WO-A-02/062215
- DE-A1- 10 239 743
- DE-A1-102005 020 569
- DE-A1-102006 020 862
- US-A- 4 127 110
- US-A1- 2004 193 020
- US-A1- 2006 241 422

## Beschreibung

Die Erfindung betrifft eine Patientendaten-Sensorvorrichtung nach dem Oberbegriff des Anspruchs 1.

Eine derartige Sensorvorrichtung ist als Hirnparameter-Sensorvorrichtung bekannt aus der DE 102 39 743 A1. Weitere Sensorvorrichtungen sind bekannt aus der WO 02/062215 A2, der US 2006/0020300 A1, der US 6 083 174, der DE 197 05 474 A1, der DE 196 38 813 C1, der DE 101 56 469 A1 und der DE 103 53 144 A1. Die bekannten Sensorvorrichtungen sind, soweit sie eine Trägerplatte mit elektronischen Komponenten aufweisen, derart baugroß, dass sie nach der Implantation vom Patienten als störend empfunden werden. Zudem besteht die Gefahr einer postoperativen Infektion.

Aus der US 2006/0241422 A1 ist eine Patientendaten-Sensorvorrichtung in Form einer In Vivo-Kapsel bekannt. Ein Patientendaten-Sensor, der zur Sensorvorrichtung gehört, hat eine in vivo einzusetzende Datenübertragungseinrichtung. Zu dieser gehören eine Antenne und eine Trägerplatte. Im Zentrum eines antennenseitigen Trägerplattenabschnitts kann bei einer der dargestellten Ausführungen ein integrierter Schaltkreis vorhanden sein. Bei in Position gefalteter Trägerplatte ist diese insgesamt von einem kapselförmigen Gehäuse umgeben. Dieses hat ein Fenster, sodass Licht auf einen bildgebenden Sensor fallen kann.

Aus der DE 10 2006 020 862 A1 ist eine Variante der Unterbringung eines implantierbaren Patientendaten-Sensors auf einer Trägerplatte bekannt. Weitere Sensoren sind bekannt aus der WO 02/062215 A2 und der DE 10 2005 020 569 A1.

Es ist daher eine Aufgabe der vorliegenden Erfindung, eine Patientendaten-Sensorvorrichtung der eingangs genannten Art derart weiterzubilden, dass sie für den Patienten verträglicher gestaltet ist.

Die Aufgabe ist erfindungsgemäß gelöst durch eine Patientendaten-Sensorvorrichtung mit den im Anspruch 1 angegebenen Merkmalen.

Erfindungsgemäß wurde erkannt, dass eine umlaufende Anordnung der Antenne eine Bauraumersparnis auf der Trägerplatte mit sich bringt, die zur Unterbringung von Bauelementen genutzt werden kann, so dass insgesamt der Bauraumbedarf der Trägerplatte, auf dem dieser elektronische Komponenten tragen muss, reduziert ist. Entsprechend kann die Trägerplatte und somit derjenige Teil der Sensorvorrichtung mit nennenswerter Bauhöhe kompakt ausgeführt sein. Die umlaufende Antenne kann ohne Weiteres so flach ausgeführt sein, dass sie im implantierten Zustand den Patienten nicht stört. Die Komponenten können auf der Trägerplatte dann so angeordnet werden, dass starre Kanten, die eine Belastung der Kopfhaut und damit eine Infektionsgefahr mit sich bringen, vermieden werden. Die Sensorvorrichtung kann insbesondere so ausgeführt sein, dass auf ein Ausfräsen des Schädelknochens zum Implantieren der Sensorvorrichtung verzichtet werden kann. Die außenumlaufende Anordnung der Antenne führt zudem zu einer Verbesserung der Datenübertragung, da der im Vergleich zum Stand der Technik große Antennendurchmesser Übertragungsvorteile bietet. Zudem ist eine Überlappung der Antenne mit den anderen elektronischen Komponenten, der zu einer Störung der Übertragung führen würde, vermieden. Im durch die Antenne vorgegebenen Außenbereich der Sensorvorrichtung muss die Kopfhaut beim Implantieren der Sensorvorrichtung nur minimal vom Schädelknochen abgehoben werden. Die Signalverbindung zwischen der Datenübertragungseinrichtung und dem Patientendaten-Sensor ist insbesondere kabelgebunden, kann prinzipiell aber auch drahtlos sein. Die Abdeckung ist zumindest in dem Bereich, wo sie die höchste Erhebung über der Trägerplatte darstellt, konvex ausgeführt. Im Normalfall ist dies ein zentraler Abschnitt der Abdeckung. Randbereiche der Abdeckung, insbesondere dort, wo diese mit der Trägerplatte verbunden ist, können auch konkav ausgestaltet sein. Die Begriffe "konvex" und "konkav" beziehen sich dabei immer auf die Gestaltung einer Außenwand der Abdeckung. Die Patientendaten-Sensorvorrichtung kann insbesondere als Hirnparameter-Sensorvorrichtung ausgeführt sein. Der Patientendaten-Sensor ist dann ein Hirnparameter-Sensor. Eine flexible Trägerlage der Trägerplatte kann aus Polyimid (PI) gefertigt sein.

Bei einer Abdeckung, die die elektronischen Komponenten auf der Trägerplatte zu beiden Seiten hin abdeckt, ist die Möglichkeit gegeben, die Trägerplatten auf beiden Seiten mit elektronischen Komponenten zu bestücken. Die Abdeckung ist zumindest abschnittsweise konvex ausgeformt. Dies reduziert den Flächenbedarf für die Trägerplatte und damit für die Sensorvorrichtung nochmals.

Eine Anordnung, bei der die Abdeckung mit einer flexiblen Trägerlage der Trägerplatte verbunden ist, erhöht die Flexibilität und damit die Form-Anpassungsfähigkeit der Sensorvorrichtung.

Wenn die Antenne als Teil einer flexiblen Antennenlage ausgeführt ist, die auf dem Randbereich der flexiblen Platine aufgebracht ist, passt sich die Sensorvorrichtung im Außenbereich im implantierten Zustand an den Schädelknochen, an dem die Sensorvorrichtung anliegt, formmäßig an. Dies erhöht den Tragekomfort der Sensorvorrichtung nochmals. Die Antennenlage ist insbesondere mit einem flexiblen Abschnitt der Trägerplatte verbunden.

Eine kreisförmige Antennenlage nach Anspruch 2 führt zu einer Symmetrisierung in Bezug auf die telemetrische Datenübertragung, was die Ankopplung der externen Datenerfassungseinheit erleichtert.

Eine Ausführung der Trägerplatte nach Anspruch 3 vereinfacht die Anbringung der Antennenlage. Alternativ kann die Trägerplatte auch einlagig ausgeführt sein.

Eine Ausführung der Antenne nach Anspruch 4 ist kostengünstig und kompakt. Alternativ kann die Antenne auch durch mindestens einen Windungsdraht, der um die elektronischen Komponenten umlaufend ausgeführt ist, gebildet sein.

Eine Beschichtung oder Einbettung nach Anspruch 5 reduziert die Materialanforderungen an die Trägerplatte. Mit der gleichen Beschichtung oder Einbettung kann auch die Abdeckung für die elektronischen Komponenten auf der Trägerplatte versehen sein. Die Beschichtung oder Abdeckung kann gleichzeitig für eine Dichtigkeit der Sensorvorrichtung sorgen, so dass kein Fluid zu den elektronischen Komponenten der Sensorvorrichtung eindringen kann. Die Beschichtung oder Abdeckung kann zudem für eine gewünschte elektrische Isolation der Antenne sorgen.

Biokompatible Materialien nach Anspruch 6 haben sich als besonders geeignet herausgestellt.

Dies gilt entsprechend für Abdeckungsmaterialien nach Anspruch 7.

Ein Stärkenverhältnis nach Anspruch 8 führt, eine gewisse Grundflexibilität des Materials der Trägerplatte vorausgesetzt, zu einer erhöhten Flexibilität der dann im Randbereich dünneren Trägerplatte. Eine derartige Ausführung kann dann zu einer insgesamt im Randbereich flexiblen Sensorvorrichtung führen, was die Anpassungsfähigkeit und damit den Tragekomfort der Sensorvorrichtung nochmals verbessert. Bei diesem Stärkenverhältnis wird die Sensorvorrichtung zudem im Randbereich, also dort, wo sie die größte laterale Erstreckung aufweist, aufgrund ihrer dann randseitig geringen Stärke vom Patienten am wenigstens als störend empfunden.

Ausführungsbeispiele der Erfindung werden nachfolgend anhand der Zeichnung näher erläutert. In diesen zeigen:
- Fig. 1: schematisch eine interne Details preisgebende Seitenansicht einer Patientendaten-Sensorvorrichtung am Beispiel einer Hirnparameter-Sensorvorrichtung;
- Fig. 2: eine Aufsicht auf die Sensorvorrichtung nach Fig. 1
- Fig. 3: eine Aufsicht auf eine weitere Ausführung einer Hirnparameter-Sensorvorrichtung;
- Fig. 4: in einer zu Fig. 1 ähnlichen und im Bereich eines Hirnparameter-Sensors gebrochenen Darstellung eine weitere Ausführung einer Hirnparameter-Sensorvorrichtung;
- Fig. 5: eine Aufsicht auf die Sensorvorrichtung nach Fig. 4 in im Vergleich zu Fig. 4 verkleinerten Maßstab;
- Fig. 6: in einer zu Fig. 4 ähnlichen Darstellung eine weitere Ausführung einer Hirnparameter-Sensorvorrichtung;
- Fig. 7: in einer zu Fig. 4 ähnlichen Darstellung eine weitere Ausführung einer Hirnparameter-Sensorvorrichtung.

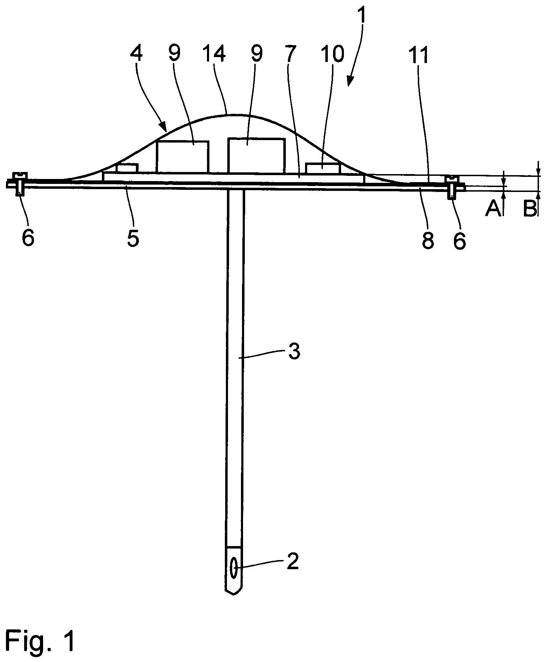

Eine Hirnparameter-Sensorvorrichtung 1 als Beispiel einer Patientendaten-Sensorvorrichtung hat einen implantierbaren Hirnparameter-Sensor 2 als Beispiel für einen Patientendaten-Sensor. Letzterer dient beispielsweise zur Messung eines Hirndrucks, beispielsweise im Parenchym oder in den Ventrikeln. Der Sensor 2 kann alternativ oder zusätzlich auch als Temperatursensor ausgeführt sein. In diesem Fall kann der Sensor 2 eine Wheatstone-Brücke aufweisen. Dabei wird insbesondere der ohmsche Widerstand der Brückendiagonale gemessen, der eindeutig der zu bestimmenden Temperatur korreliert ist. Über einen Katheterschlauch 3, der starr oder flexibel ausgeführt sein kann, ist der Sensor 2 mit einer Trägerbaugruppe 4 verbunden. Der Katheterschlauch 3 ist aus Kunststoffmaterial. Im Katheterschlauch 3 verlaufen in der Fig. 1 nicht dargestellte Signal- und Versorgungsleitungen einerseits zur Energieversorgung des Sensors 2 und andererseits zur Signalübermittlung, insbesondere zur Übermittlung von Mess- und Steuersignalen. Prinzipiell ist es möglich, anstelle des Katheterschlauchs 3 auch eine drahtlose Verbindung zwischen dem Sensor 2 und der Trägerbaugruppe 4 zu schaffen.

Die Trägerbaugruppe 4 wird auf einem in der Zeichnung nicht dargestellten Schädelknochen eines Patienten aufgesetzt und fixiert. Der Katheterschlauch 3 mit dem Sensor 2 wird durch eine entsprechende Bohrung durch den Schädelknochen hindurchgeführt, bis die Messposition erreicht ist. Die Messposition kann dabei subdural oder epidural erfolgen.

Im implantierten Zustand ist dem Schädelknochen zugewandt eine flexible Platine 5 mit einer Stärke von etwa 1 mm. Die flexible Platine 5 hat die Form einer runden Scheibe. Über Schrauben 6 ist die Platine 5 am Schädelknochen fixierbar. Die Schrauben 6 können durch Fixationslaschen in der flexiblen Platine 5 hindurchgeführt sein. Die flexible Platine 5 ist Teil einer Trägerplatte der Trägerbaugruppe 4. Die Platine 5 hat eine Stärke, die in der Fig. 1 mit A bezeichnet ist.

Auf die flexible Platine 5 aufgesetzt und mit dieser verbunden ist eine starre Platine 7, auf der elektronische Bauteile aufgebracht sind.

Die starre Platine 7 hat die Form einer runden Scheibe, deren Durchmesser kleiner ist als derjenige der flexiblen Platine 5. Die beiden Platinen 5, 7 sind konzentrisch zueinander angeordnet, so dass die flexible Platine 5 in einem umlaufenden, ringförmigen Randbereich 8 über die starre Platine 7 übersteht. Die starre Platine 7 hat eine Stärke, die in der Fig. 1 mit B bezeichnet ist.

Die beiden Platinen 5, 7 stellen zwei Trägerlagen der Trägerplatte der Sensorvorrichtung 1 dar. Die flexible Platine 5 ist insbesondere auf die starre Platine 7 auflaminiert. Die flexible Platine 5 stellt eine PCB-(Printed Circuit Board-) Platine dar. Die starre Platine 7 ist aus dem Material FR4.

Für das Stärkenverhältnis A/B gilt: A ≤ 0,5 B. Im Randbereich 8 hat die Trägerplatte 5, 7 also eine Plattenstärke A, die höchstens die Hälfte der in der gleichen Richtung gemessenen Stärke B des die elektronischen Komponenten 9, 10 tragenden Bereichs der Trägerplatte 5, 7 beträgt.

Zu den elektronischen Bauteilen gehören bauhohe SMD-Bauteile 9, die nahe des Zentrums der starren Platine 7 untergebracht sind. Im Randbereich 8 der starren Platine 7 sind weitere, flache elektronische Bauteile 10 auf der starren Platine 7 angeordnet. Die Bauteile 9, 10 stellen elektronische Komponenten der Sensorvorrichtung 1 dar. Diese Bauteile 9, 10 dienen zur elektrischen Versorgung des Sensors 2 sowie zum telemetrischen Datenaustausch zwischen dem Sensor 2 und einer externen Auslese- und Steuereinrichtung, die in der Zeichnung nicht dargestellt ist. Die Auslese- und Steuereinrichtung stellt eine externe Datenerfassungseinheit dar. Aufgrund ihrer Telemetriefunktion wird die Trägerbaugruppe 4 auch als Telemetrieeinheit bzw. Datenübertragungseinrichtung bezeichnet. Zudem ist die Telemetrieeinheit für die Konditionierung der vom Sensor 2 erfassten Messdaten zuständig. Diese Signalkonditionierung erfolgt in einer anwendungsspezifisch integrierten Schaltung (application specific integrated circuit, ASIC). Ein Sensortyp des Sensors 2 ist über ein im Katheterschlauch verlaufendes Mikrokabel direkt mit dem ASIC verbunden. Der ASIC dient unter anderem als Multiplexer, d. h. als Schaltnetzwerk bzw. als Umschalter, als A/D-Wandler und als serielle Schnittstelle. Bei der Signal- bzw. Messwertkonditionierung werden mit dem Sensor 2 eine Drucksignalspannung und ein Brückendiagonalwiderstand der Wheatstone-Brücke zyklisch gemessen. Dazu werden Sensorleitungen auf der Trägerplatte 5, 7 mit Hilfe der Multiplex-Funktion des ASIC periodisch umgeschaltet. Die nach dem Multiplexen abgefragten analogen Signalwerte werden an den A/D-Wandler weitergeleitet und dort in ein digitales Messdaten-Signal gewandelt. Dieses wird über die serielle Schnittstelle des ASIC zur Weiterverarbeitung zur Verfügung gestellt.

Die Telemetrieeinheit kann als passiver Transponder ausgeführt sein, benötigt also nicht zwingend eine eigene Energieversorgung.

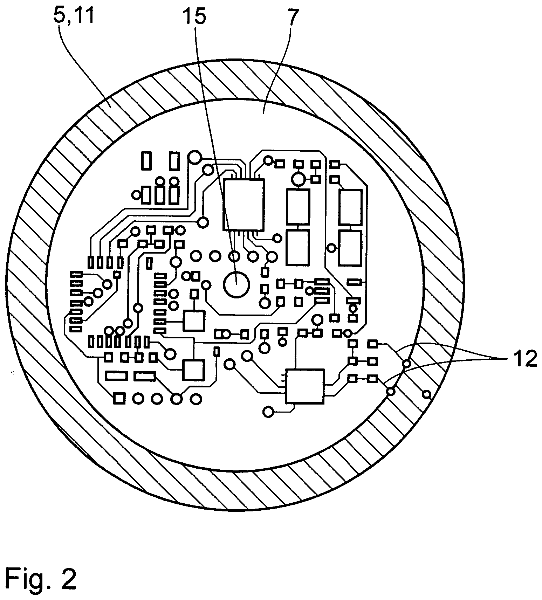

Auf dem Randbereich 8 der flexiblen Platine 5 aufgebracht ist eine dünne, ringförmige Antennenlage, die eine um die flexible Platine 5 umlaufende Antennenspule 11 (vgl. Fig. 2) vorgibt. Die Antennenspule 11 liegt also auf dem die Trägerplatte 5, 7 umlaufenden Randbereich 8 auf. Die Antennenspule 11 ist also flächig mit der Trägerplatte 5, 7 verbunden. Die Antennenspule 11 ist als gedruckte Schaltung ausgeführt. Die Antennenspule bzw. Antennenlage 11 ist zwischen den beiden durch die Platinen 5, 7 gebildeten Trägerlagen der Trägerplatte angeordnet. Die Antennenspule 1 kann auch mehr als eine Lage aufweisen. Bonding-Kontakte 12 verbinden die Antennenspule 11 mit zugeordneten Bauteilen auf der starren Platine 7. Da die Antennenspule 11 auf der flexiblen Platine 5 aufgebracht ist, ist dieser äußere Antennenbereich der Sensorvorrichtung 1 dünn und flexibel ausgeführt.

Über die Antennenspule 11 findet eine telemetrische Datenübertragung zwischen der Sensorvorrichtung 1 und der externen Auslese- und Steuereinrichtung statt.

Im implantierten Zustand ist die Sensorvorrichtung 1 unter der Kopfhaut des Patienten untergebracht. Zur Kopfhaut hin wird die Sensorvorrichtung 1 von einer Abdeckung in Form einer flexiblen Membran 14 abgedeckt. Die Außenwand der Membran 14 hat eine asphärische und konvexe Form, ist gegenüber einer Kugelform also deutlich abgeflacht. Insgesamt ist der Teil der Sensorvorrichtung 1, der im implantierten Zustand auf dem Schädelknochen angeordnet ist, so flach, dass keine unerwünscht hohen Spannungen der Kopfhaut auftreten. Die flexible Membran 14 führt zudem dazu, dass die Sensorvorrichtung 1 auch im Bereich der Bauteile 9, 10 auf der der Kopfhaut zugewandten Seite nicht scharfkantig ist.

Zentral in der Trägerplatte 5, 7 ist ein Anschluss 15 zur mechanischen und elektrischen Ankopplung des Katheterschlauchs 3 an die Trägerplatte 5, 7 vorgesehen.

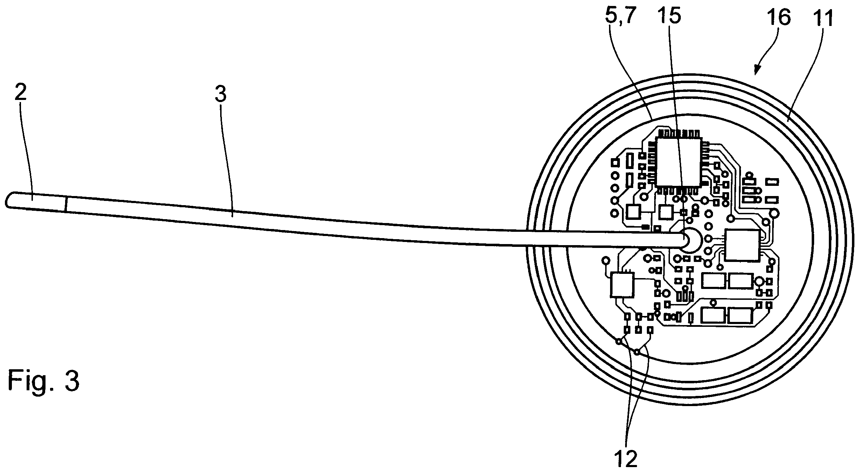

Fig. 3 zeigt eine weitere Ausführung einer Hirnparameter-Sensorvorrichtung 16. Komponenten, die denjenigen entsprechen, die vorstehend schon unter Bezugnahme auf die Fig. 1 und 2 erläutert wurden, tragen die gleichen Bezugsziffern und werden nicht nochmals im Einzelnen diskutiert.

Bei der Ausführung der Sensorvorrichtung 16 ist der Katheterschlauch 3 seitlich von der Trägerplatte 5, 7 weggeführt, verläuft also benachbart zur Trägerplatte 5, 7 zunächst in einer von dieser vorgegebenen Trägerebene. Der Katheterschlauch 3 ist so flexibel, dass er im implantierten Zustand in etwa S-förmig gebogen durch eine entsprechend ausgeführte Öffnung in der Schädeldecke durch diese insbesondere für eine epidurale Messung hindurchgeführt wird. Der seitlich herausgeführte Katheterschlauch 3 unterbricht bei der Ausführung nach Figur 3 die Antennenspule 11 nicht, sondern ist in der Orientierung nach Figur 3 dem Betrachter zugewandt zur Antennenspule 11 versetzt, also oberhalb der Antennenspule 11 verlegt.

Bei den Ausführungen nach den Fig. 1 bis 3 ist die flexible Platine 5 im Durchmesser etwa 20 % größer als die starre Platine 7. Die starre Platine 7 kann, was ihren absoluten Durchmesser angeht, noch kleiner ausgeführt sein als in den Fig. 1 bis 3, so dass auch ein noch größeres Durchmesserverhältnis zwischen der flexiblen Platine 5 und der starren Platine 7 möglich ist. Die Verkleinerung des Durchmessers der starren Platine 7 kann erreicht werden durch eine beidseitige Bestückung der starren Platine 7 mit Bauteilen 9, 10. In diesem Fall weist die flexible Platine 5 an den Orten auf der Rückseite der starren Platine 7, wo Bauteile 9, 10 untergebracht sind, entsprechende Aussparungen auf.

Im Falle, dass die starre Platine 7 beiderseits mit Bauteilen 9, 10 bestückt ist, resultiert, was die Trägerbaugruppe 4 in ihrer Querschnittskontur entsprechend Fig. 1 angeht, eine weitgehend um die Ebene der Trägerplatte 5, 7 spiegelsymmetrische und in etwa linsenförmige Ausführung mit zwei die Trägerplatte 5, 7 und die dort beiderseits aufgebrachten Bauteile 9, 10 abdeckenden flexiblen Membranen 14.

Anstelle der Membran 14 kann bei der Ausführung nach den Fig. 1 bis 3 auch eine ebenfalls konvex geformte Abdeckung aus Keramik oder Titan vorgesehen sein.

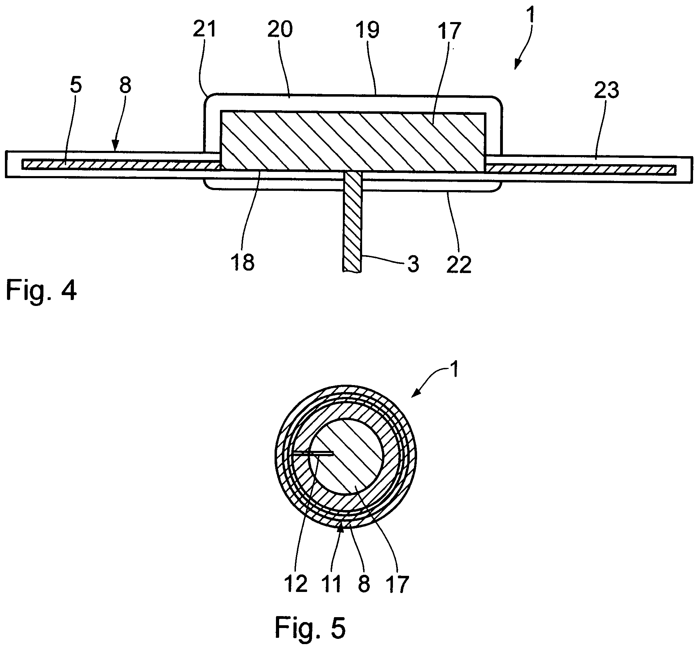

Fig. 4 und 5 zeigen eine weitere Ausführung einer Hirnparameter-Sensorvorrichtung 1. Komponenten, die denjenigen entsprechen, die vorstehend unter Bezugnahme auf die Fig. 1 bis 3 bereits erläutert wurden, tragen die gleichen Bezugsziffern und werden nicht nochmals im Einzelnen diskutiert.

Bei der Ausführung nach Fig. 4 sind die starre Platine sowie die Bauteile der Ausführung nach den Fig. 1 bis 3 zu einem inneren Elektronikgehäuse 17 zusammengefasst, dass auch als Elektronik-Board bezeichnet ist. Das Elektronikgehäuse 17 hat die Form eines flachen Zylinders mit Dimensionsverhältnissen von Zylinderhöhe und Zylinderdurchmesser, die einem maßstäblich verkleinerten Eishockey-Puck vergleichbar sind.

Die flexible Platine 5, die auch als Antennen-Board bezeichnet ist, steht seitlich über eine Bodenfläche 18 des Elektronikgehäuses 17 über. Die Antennenspule 11 ist wie bei der Ausführung nach den Fig. 1 bis 3 auf der flexiblen Platine 5 aufgebracht und in der Fig. 4 nicht dargestellt. Die Antennenspule 11 kann in Form von auf die flexible Platine 5 aufgedruckten Windungen oder in Form von separaten Windungsdrähten ausgeführt sein. Dieser Überstand der flexiblen Platine 5 ist entsprechend dem Überstand der flexiblen Platine über die starre Platine bei der Ausführung nach den Fig. 1 bis 3. Das innere Elektronikgehäuse 17 ist umgeben von einem äußeren Abdeckungsgehäuse 19, das die Funktion der Membran 14 bei der Ausführung nach den Fig. 1 bis 3 hat.

Das äußere Abdeckungsgehäuse 19 hat eine schalenförmige Abdeckung 20 aus Keramik oder Titan, die komplementär zum über die flexible Platine 5 in der Fig. 4 nach oben überstehenden Abschnitt des Elektronikgehäuses 17 ausgeführt ist. Eine das innere Elektronikgehäuse 17 nach oben hin abdeckende Deckwand der schalenförmigen Abdeckung 20 geht über eine ringförmige abgerundete Kante 21 in eine hülsenartige Seitenwand der schalenförmigen Abdeckung 20 über, die sich bis zur flexiblen Platine 5 erstreckt.

Neben der in der Fig. 4 oberen schalenförmigen Abdeckung 20 kann das Abdeckungsgehäuse 19 noch einen in der Fig. 4 unten dargestellten runden Gegendeckel 22, ebenfalls aus Keramik oder Titan, aufweisen. Letzterer hat eine zentrische Durchgangsöffnung für den Hirnparameter-Sensor 2. Zwischen der schalenförmigen Abdeckung 20 und dem Gegendeckel 22 verbleibt rund um die Zylinder-Mantelwand des Elektronikgehäuses 17 ein ringförmiger Durchgang für die flexible Platine 5.

Die flexible Platine 5 ist eingebettet in eine biokompatible Einbettung 23, die bei der Ausführung nach den Fig. 4 und 5 als Beschichtung der flexiblen Platine aus Silikon, Silikonkautschuk oder Polyurethan ausgeführt ist. Auch eine Beschichtung mit Parylene ist möglich. Die Einbettung 23 sorgt dafür, dass ein Patientenkontakt mit der Sensorvorrichtung 1 ausschließlich über das Abdeckgehäuse 19, über die Einbettung 23 und über den Hirnparameter-Sensor 2 möglich ist. Die Einbettung 23 dichtet die flexible Platine 5 gegen das Abdeckungsgehäuse 19 ab. Die biokompatible Einbettung 23, die in der Fig. 4 als Schicht unterhalb der flexiblen Platine 5 angebracht ist, dichtet gegen den Katheterschlauch 3 des Sensors 2 ab.

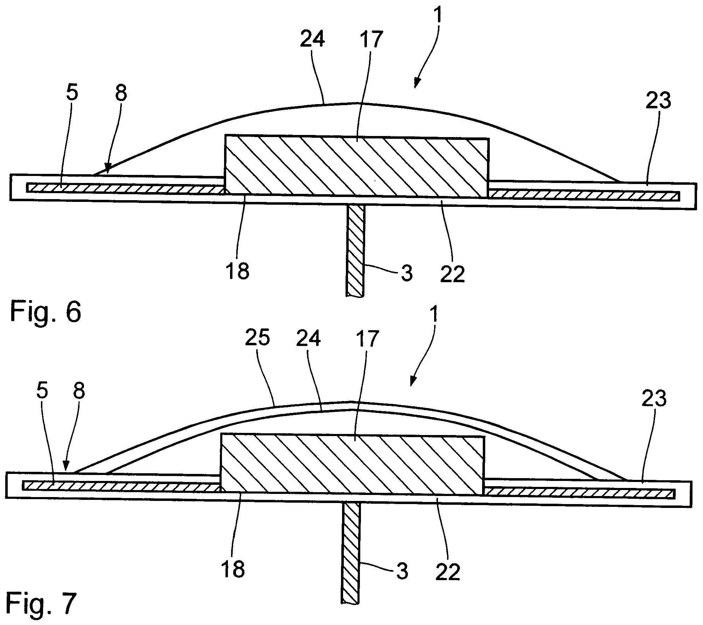

Anhand der Fig. 6 wird nachfolgend eine weitere Ausführung einer Hirnparameter-Sensorvorrichtung erläutert. Komponenten, die denjenigen entsprechen, die vorstehend unter Bezugnahme auf die Ausführungen nach den Fig. 1 bis 5 beschrieben wurden, tragen die gleichen Bezugsziffern und werden nicht nochmals im Einzelnen diskutiert.

Die Sensorvorrichtung 1 nach Fig. 6 hat im Vergleich zur Sensorvorrichtung nach den Fig. 4 und 5 keine komplementär zum Elektronikgehäuse 17 ausgebildete schalenförmige Abdeckung, sondern eine konvexe Abdeckung 24 aus Keramik oder Titan. Letztere überwölbt den in der Fig. 6 nach oben über die flexible Platine 5 hinausragenden Abschnitt des Elektronikgehäuses 17 und dichtet randseitig gegen die biokompatible Einbettung 23 auf der flexiblen Platine 5 ab.

Anhand der Fig. 7 wird nachfolgend eine weitere Ausführung einer Hirnparameter-Sensorvorrichtung 1 beschrieben. Komponenten, die denjenigen entsprechen, die vorstehend unter Bezugnahme auf die Ausführungen nach den Fig. 1 bis 6 erläutert wurden, tragen die gleichen Bezugsziffern und werden nicht nochmals im Einzelnen diskutiert.

Im Unterschied zur Ausführung nach Fig. 6 weist die Abdeckung 24 nach Fig. 7 eine zusätzliche äußere Beschichtung 25 aus biokompatiblem Material, also beispielsweise ebenfalls aus Silikon oder Polyurethan, auf. Die Beschichtung 25 dichtet randseitig gegen die Einbettung 23 der flexiblen Platine 5 ab. Alternativ zu einer einseitigen Beschichtung der konvexen Abdeckung 24 kann letztere auch in biokompatibles Material, also beispielsweise in Silikon oder Polyurethan, eingebettet sein und vollständig von dieser Einbettung umhüllt sein, wie dies vorstehend am Beispiel der flexiblen Platine 5 erläutert wurde.

## Patentansprüche

1. Patientendaten-Sensorvorrichtung (1, 16)
- mit einem implantierbaren Patientendaten-Sensor (2),
- mit einer implantierbaren Datenübertragungseinrichtung (4), die mit dem Patientendaten-Sensor (2) in Signalverbindung steht,
- wobei die Datenübertragungseinrichtung (4)
- - eine Antenne (11) zur telemetrischen Datenübertragung an eine externe Datenerfassungseinheit,
- - eine Trägerplatte (5, 7) für elektronische Komponenten (9, 10) aufweist,
- wobei die Antenne (11) in einem Randbereich (8) der Trägerplatte (5, 7) angeordnet und um diese und um die elektronischen Komponenten (9, 10) umlaufend ausgeführt ist,
- wobei die elektronischen Komponenten (9, 10) auf der Trägerplatte (5, 7) zumindest nach einer Seite hin von einer Abdeckung (14) abgedeckt werden,
**dadurch gekennzeichnet, dass**
- die Trägerplatte (5, 7) zwei Trägerlagen aufweist, die durch eine flexible Platine (5) und eine darauf aufgesetzte und mit dieser verbundenen starren Platine (7) gebildet sind, wobei die elektronischen Komponenten (9, 10) auf der starren Platine (7) aufgebracht sind,
- die flexible Platine (5) in dem umlaufenden Randbereich (8) über die starre Platine (7) übersteht,
- die mindestens eine Abdeckung (14) mit der flexiblen Platine (5) der Trägerplatte (5, 7) verbunden ist,
- die Antenne als Teil einer flexiblen Antennenlage (11) ausgeführt ist, die auf dem Randbereich (8) der flexiblen Platine (5) aufgebracht ist, um die Sensorvorrichtung (1, 16) im Außenbereich im implantierten Zustand an den Schädelknochen, an dem die Sensorvorrichtung (1, 16) anliegt, formmäßig anzupassen,
- die Patientendaten-Sensorvorrichtung (1, 16) als Hirnparameter-Sensorvorrichtung ausgeführt ist.

2. Sensorvorrichtung nach Anspruch 1, **dadurch gekennzeichnet, dass** die Antenne (11) kreisförmig ausgeführt ist.

3. Sensorvorrichtung nach Anspruch 1 oder 2, **dadurch gekennzeichnet, dass** die Trägerplatte (5, 7) zweischichtig mit zwei Trägerlagen ausgeführt ist, zwischen denen die Antennenlage (11) angeordnet ist.

4. Sensorvorrichtung nach einem der Ansprüche 1 bis 3, **dadurch gekennzeichnet, dass** die Antenne (11) als gedruckte Schaltung ausgeführt ist.

5. Sensorvorrichtung nach einem der Ansprüche 1 bis 4, **dadurch gekennzeichnet, dass** die Trägerplatte (5, 7) dort, wo sie von außen her zugänglich wäre, von einer Beschichtung oder Einbettung (23) aus einem biokompatiblen Material abgedeckt ist.

6. Sensorvorrichtung nach Anspruch 5, **gekennzeichnet durch** eine Beschichtung oder Einbettung (23) aus Silikon, Silikonkautschuk, Parylene oder aus Polyurethan.

7. Sensorvorrichtung nach einem der Ansprüche 1 bis 6, **dadurch gekennzeichnet, dass** die Abdeckung (24) aus Keramik oder Titan gefertigt ist.

8. Sensorvorrichtung nach einem der Ansprüche 1 bis 7, **dadurch gekennzeichnet, dass** die Trägerplatte (5, 7) in einem die Antenne (11) tragenden Randbereich (8) eine Plattenstärke (A) hat, die höchstens die Hälfte einer in der gleichen Richtung gemessenen Stärke (B) eines die elektronischen Komponenten (9, 10) tragenden Bereichs (7) der Trägerplatte (5, 7) beträgt.

## Claims

1. Patient data sensor device (1; 16)
- with an implantable patient data sensor (2),
- with an implantable data transmission mechanism (4), which is in signal connection with the patient data sensor (2),
- wherein the data transmission mechanism (4)
- - has an antenna (11) for telemetric data transmission to an external data collection unit,
- - a carrier plate (5, 7) for electronic components (9, 10),
- wherein the antenna (11) is arranged in an edge region (8) of the carrier plate (5, 7) and is configured running around the latter and around the electronic components (9, 10),
- wherein the electronic components (9, 10) on the carrier plate (5, 7) are covered at least to one side by a cover (14),
**characterised in that**
- the carrier plate (5, 7) has two carrier layers, which are formed by a flexible board (5) and a rigid board (7), which is placed on said flexible board (5) and connected thereto, wherein the electronic components (9, 10) are attached to said rigid board (7),
- the flexible board (5) in a peripheral edge region (8) projects over the rigid board (7),
- the at least one cover (14) is connected to the flexible board (5) of the carrier plate (5, 7),
- the antenna is configured as part of a flexible antenna layer (11), which is located on the edge region (8) of the flexible board (5) to adapt, in terms of shape, the sensor device (1, 16) in the outer region, when implanted, to the cranial bone, on which the sensor device (1, 16) rests,
- the patient data sensor device (1, 16) is configured as a brain parameter sensor device.

2. Sensor device according to claim 1, **characterised in that** the antenna (11) is circular.

3. Sensor device according to claim 1 or 2, **characterised in that** the carrier plate (5, 7) is configured in two layers with two carrier layers, between which the antenna layer (11) is arranged.

4. Sensor device according to any one of claims 1 to 3, **characterised in that** the antenna (11) is configured as a printed circuit.

5. Sensor device according to any one of claims 1 to 4, **characterised in that** the carrier plate (5, 7) is covered, where it would be accessible from the outside, by a coating or embedding (23) made of a biocompatible material.

6. Sensor device according to claim 5, **characterised by** a coating or embedding (23) made of silicone, silicone rubber, parylene or of polyurethane.

7. Sensor device according to any one of claims 1 to 6, **characterised in that** the cover (24) is produced from ceramic or titanium.

8. Sensor device according to any one of claims 1 to 7, **characterised in that** the carrier plate (5, 7), in an edge region (8) carrying the antenna (11), has a plate thickness (A), which is at most half a thickness (B) measured in the same direction of a region (7) of the carrier plate (5, 7) carrying the electronic components (9, 10).

## Revendications

1. Dispositif capteur de données de patients (1, 16)
- avec un capteur implantable pour des données de patients (2),
- avec un dispositif implantable de transmission de données (4) qui est en liaison de signalisation avec le capteur de données de patients (2),
- le dispositif de transmission de données (4) présentant
- - une antenne (11) pour la transmission télémétrique de données à une unité externe d'acquisition de données,
- - une plaque de support (5, 7) pour des composants électroniques (9, 10),
- l'antenne (11) étant disposée dans une périphérie (8) de la plaque de support (5, 7) et étant réalisée sur toute la périphérie de celle-ci ainsi que des composants électroniques (9, 10),
- les composants électroniques (9, 10) étants couverts sur la plaque de support (5, 7), au moins vers un côté, par un couvercle (14),
**caractérisé en ce que**
- la plaque de support (5, 7) présente deux couches de support qui sont formées par une platine flexible (5) et une platine rigide (7) posée au-dessus de et connectée à celle-ci, les composants électroniques (9, 10) étant appliqués sur la platine rigide (7),
- la platine flexible (5) fait saillie de la platine rigide (7) dans la périphérie (8),
- l'au moins un couvercle (14) est connecté à la platine flexible (5) de la plaque de support (5, 7),
- l'antenne est réalisée en tant que partie d'une couche flexible d'antenne (11), qui est appliquée sur la périphérie (8) de la platine flexible (5), pour adapter le dispositif capteur (1, 16) dans la zone extérieur en l'état implanté à la forme de l'os crânien sur lequel le dispositif capteur (1, 16) s'appuie,
- le dispositif capteur de données de patients (1, 16) est réalisé comme dispositif capteur de paramètres cérébraux.

2. Dispositif capteur selon la revendication 1, **caractérisé en ce que** l'antenne (11) est réalisée en forme circulaire.

3. Dispositif capteur selon la revendication 1 ou 2, **caractérisé en ce que** la plaque de support (5, 7) est réalisée de deux couches avec deux couches de support entre lesquelles la couche d'antenne (11) est disposée.

4. Dispositif capteur selon l'une quelconque des revendications 1 à 3, **caractérisé en ce que** l'antenne (11) est réalisée comme circuit imprimé.

5. Dispositif capteur selon l'une quelconque des revendications 1 à 4, **caractérisé en ce que** la plaque de support (5, 7) est couvert par un revêtement ou un encastrement (23) d'un matériel biocompatible à l'endroit où elle serait accessible de l'extérieur.

6. Dispositif capteur selon la revendication 5, **caractérisé par** un revêtement ou un encastrement (23) de silicone, caoutchouc de silicone, parylène ou de polyuréthane.

7. Dispositif capteur selon l'une quelconque des revendications 1 à 6, **caractérisé en ce que** le couvercle (24) est fabriqué en céramique ou en titane.

8. Dispositif capteur selon l'une quelconque des revendications 1 à 7, **caractérisé en ce que** la plaque de support (5, 7), dans une périphérie (8) portant l'antenne (11), présente une épaisseur de la plaque (A) qui s'élève au maximum à la moitié d'une épaisseur (B) d'une zone (7) de la plaque de support portant les composants électroniques (9, 10), mesurée dans la même direction.
